# EUROPÄISCHE PATENTSCHRIFT

(11) **EP 3 160 326 B1**
(45) Veröffentlichungstag und Bekanntmachung des Hinweises auf die Patenterteilung: **23.10.2019**
(21) Anmeldenummer: 15723834.6
(22) Anmeldetag: 07.05.2015
(51) Int. Cl.: A61B 3/00, A61B 3/032, A61B 3/028, A61B 3/036

(54) **VERFAHREN FÜR EINE SEHSCHÄRFEBESTIMMUNG**
METHOD FOR DETERMINING VISUAL ACUITY
PROCÉDÉ DE DÉTERMINATION DE L'ACUITÉ VISUELLE

(30) Priorität: 25.06.2014 DE 102014009459
(43) Veröffentlichungstag der Anmeldung: 03.05.2017
(73) Patentinhaber: Rodenstock GmbH, 80687 München (DE)
(72) Erfinder: SCHUBART, Johannes, 82041 Deisenhofen (DE); SEITZ, Peter, 71272 Renningen (DE); PERANI, Kilian, 80333 München (DE)
(74) Vertreter: Müller-Boré & Partner Patentanwälte PartG mbB
(86) Internationale Anmeldenummer: PCT/EP2015/000935
(87) Internationale Veröffentlichungsnummer: WO 2015/197149

(56) Entgegenhaltungen:
- EP-A1- 2 561 799
- WO-A1-2008/064379
- US-A1- 2003 218 721
- US-A1- 2008 259 278
- US-A1- 2012 212 706
- US-B1- 6 238 049

## Beschreibung

Die Erfindung betrifft ein Verfahren zum Bereitstellen von Sehzeichen, ein Verfahren zum Bestimmen von optischen Korrekturweiten eines Probanden, ein Computerprogrammprodukt und einen mobilen Computer.

Zur Bestimmung von Korrektionswerten eines fehlsichtigen Probanden findet die Refraktion Anwendung. Dem Probanden werden dabei Sehzeichen in verschiedenen Ausprägungen gezeigt. Diese dienen dabei der Bestimmung des Visus, also der Sehschärfe, und des Kontrastvermögens des Probanden. Mittels zusätzlicher Sehtests kann darüber hinaus das Akkommodationsgleichgewicht, das Farbensehen oder der Binokularstatus geprüft werden.

Zur Durchführung dieser Tests werden entweder klassische Prüftafeln verwendet, die in einer vorbestimmten Entfernung vom Probanden aufgestellt werden müssen, oder es werden teure Refraktionseinheiten verwendet, die die Sehtests über manuell zu kalibrierende Projektoren darstellen. Die Geräte sind dabei fest installiert. Insbesondere bei Sehprüfungen für die Ferne, also für Sehprüfungen ab einer Prüfentfernung von mindestens einem Meter, insbesondere von mindestens vier Metern, oder bei Sehprüfungen bei einer verkürzten Prüfdistanz, sind zum Beispiel bei Hausbesuchen oder bei Veranstaltungen nur mit hohem Aufwand realisierbar, oder durch Mitführung von mehreren Prüftafeln. Einige Sehtests, wie zum Beispiel ein Rot-Grün Test, sind nicht mehr als Prüftafel erhältlich. Andere Sehtests, wie z.B. die sogenannten Vistech Tafeln und die Pelli Robson Charts, werden nicht mehr als Prüftafeln hergestellt und sind deswegen nur sehr schwer, nur als Restbestände oder gar nicht mehr erhältlich.

Grundsätzlich ist die subjektive Refraktion in ihrem Ablauf vielfach in der Fachliteratur beschrieben. Auch zur Nutzung von Testtafeln und Refraktionseinheiten gibt es Handbücher und Veröffentlichungen. Bei der Refraktion unterscheidet man die Sehschärfebestimmung für die Nähe im Abstand von ca. 25 cm bis ca. 60 cm und die Sehschärfebestimmung für die Ferne, die normalerweise im Abstand von mindestens vier Metern durchgeführt wird. Hat ein Patient oder Kunde eine starke Fehlsichtigkeit oder besondere Bedürfnisse, kann auch eine Sehprüfung bei verkürzter Prüfdistanz durchgeführt werden.

Fest installierte Refraktionseinheiten haben einen hohen Platzbedarf, weisen hohe Anschaffungskosten von 10 000 bis 20 000 EUR auf und können nur mit einem sehr hohen Aufwand transportiert und mobil genutzt werden.

Prüftafeln sind zwar etwas handlicher als Refraktionseinheiten, jedoch sind nicht für alle Sehtests Prüftafeln erhältlich. Ein weiterer Nachteil der Prüftafeln ist deren begrenzte Haltbarkeit und Abnutzungserscheinungen beim Transport.

Beide bislang bekannten Verfahren zum Durchführen eines Sehtests weisen den Nachteil auf, dass sie für eine vorbestimmte Prüfentfernung manuell kalibriert werden müssen oder speziell für diese hergestellt werden. Wird diese Prüfentfernung geändert oder kann diese Prüfentfernung nicht eingehalten werden, muss der Prüfer die erzielten Ergebnisse beim Ablesen der Sehzeichen umrechnen, um entsprechende Angaben zum Visus etc. zu erhalten, die für den Sehtest mit abgeänderter Prüfentfernung gelten.

Dokument US 2003/0218721 A1 betrifft ein System und ein Verfahren um dabei zu helfen, einen Benutzer möglichst günstig vor einem Computerbildschirm anzuordnen, um die Belastung für die Augen des Benutzers zu minimieren. Dabei wird mittels eines Distanzsensors ein Sollabstand zwischen Benutzer und Computerbildschirm bestimmt und überwacht. Der Distanzsensor kann als ein Echolot mit Hochfrequenzschallwellen ausgebildet sein.

Dokument WO 2008/064379 A1 betrifft eine Vorrichtung zum Erfassen einer Lese-Sehschärfe einer Testperson. Hierbei werden zwei Videokameras zum Ermitteln einer Prüfentfernung der Testperson mittels eines Stereobild-Verfahrens verwendet.

Dokument US 2012/0212706 A1 betrifft ein Verfahren und ein System zum Durchführen eines Sehtests mittels eines mobilen Computergeräts. Hierbei wird eine Prüfentfernung entweder vorbestimmt oder muss vom Benutzer manuell eingegeben werden.

Der Erfindung liegt die Aufgabe zugrunde, eine vereinfachte Sehschärfebestimmung für die Ferne zu ermöglichen. Der Erfindung liegt insbesondere die Aufgabe zugrunde, eine Sehschärfebestimmung für die Ferne für eine variable Prüfentfernung und/oder für einen mobilen Einsatz zu ermöglichen.

Diese Aufgabe wird durch die Gegenstände der unabhängigen Ansprüche gelöst. Bevorzugte Ausführungsformen sind die Gegenstände der abhängigen Ansprüche.

Ein Aspekt betrifft ein Verfahren zum Bereitstellen von Sehzeichen für eine Sehschärfebestimmung für die Ferne, mit den Schritten:
- Bereitstellen einer Probandenposition für einen Probanden,
- Bereitstellen einer Darstellungsoberfläche an einer Darstellungsposition,
- Detektieren einer individuellen Prüfentfernung zwischen der Probandenposition und der Darstellungsposition,
- Bestimmen einer individuellen Sollgröße für Sehzeichen, die gemäß einer vorbestimmten Regel auf die detektierte individuelle Prüfentfernung abgestimmt sind,
- Bereitstellen von digitalen Sehzeichenbilddaten, die auf die individuelle Sollgröße skalierte Sehzeichen enthalten, und
- Darstellen der digitalen Sehzeichenbilddaten auf der Darstellungsoberfläche.

Durch das Verfahren werden Sehzeichen, sogenannte Optotypen, bereitgestellt, deren Größe auf die individuelle Prüfentfernung abgestimmt ist. Dabei ist die individuelle Prüfentfernung dynamisch und/oder variabel.

Zum Bereitstellen der Probandenposition wird eine Prüfposition festgelegt, an der sich ein Proband beim Durchführen der Sehschärfebestimmung als Sehtest aufhalten soll. Dazu kann z.B. eine Sitzgelegenheit zur Verfügung gestellt werden, auf der der Proband beim Durchführen des geplanten Sehtests sitzen kann. Die Probandenposition kann auch durch Bestimmung eines anderen Aufenthalts für den Probanden bereitgestellt werden, zum Beispiel durch Markierung eines Stehplatzes oder der Auswahl eines Liegeplatzes, zum Beispiel für bettlägerige Probanden. Das Bereitstellen der Probandenposition entspricht somit der Bestimmung eines Aufenthaltsorts, an dem sich der Proband bei Durchführung des geplanten Sehtests aufhalten kann. Sehtests werden regelmäßig mit Hilfe einer Refraktionsbrille durchgeführt, die der Proband beim Sehtest trägt. Bei Refraktionsbrillen können unterschiedliche Prüfgläser vorgesteckt werden, bis die für den Probanden subjektiv empfundene beste Korrektion der Fehlsichtigkeit gefunden wurde. Man spricht hierbei von der subjektiven Refraktion.

Die Probandenposition kann genauer definiert werden als Position der Refraktionsbrille bei Durchführung des Tests, also auf der Nase des Probanden. Alternativ kann die Probandenposition auch als Augenposition des Probanden beim Test definiert werden.

Als Darstellungsoberfläche wird vorzugsweise eine Darstellungsoberfläche ausgewählt, auf der digitale Bilddaten unmittelbar angezeigt werden können, wie zum Beispiel ein Monitor, eine Leinwand, eine Projektionswand, ein Fernsehbildschirm, ein Display eines mobilen Computergeräts, etc.

Durch das Festlegen der Probandenposition und der Darstellungsposition wird die individuelle Prüfentfernung festgelegt und definiert, die der Entfernung der Probandenposition von der Darstellungsposition entspricht. Die individuelle Prüfentfernung kann z.B. automatisch detektiert werden.

In Abhängigkeit der detektierten individuellen Prüfentfernung wird die individuelle Sollgröße für Sehzeichen bestimmt, die sich in einem Abstand vom Probanden befinden, der der individuellen Prüfentfernung entspricht. Die zu bestimmende Sollgröße der Sehzeichen kann eine Fläche, eine Höhe, eine Breite und/oder einen Durchmesser der Sehzeichen enthalten. Da zur Durchführung eines Sehtests in der Regel eine Mehrzahl von Sehzeichen verwendet werden, die unterschiedliche Größen aufweisen können, können bei diesem Schritt auch unterschiedliche individuelle Sollgrößen bestimmt werden. Dabei kann zum Beispiel für jedes Sehzeichen eine spezielle, dem Sehzeichen zugeordnete individuelle Sollgröße des Sehzeichens bestimmt werden. Alternativ oder zusätzlich kann als Sollgröße auch lediglich ein Skalierungsfaktor für digitale Bilder festgelegt werden, zum Beispiel von vorab bereitgestellten digitalen Bildern von Prüftafeln, die Sehzeichen aufweisen und zur Durchführung eines Sehtest ausgebildet und vorgesehen sind. Diese digitalen Bilder von Sehzeichen können zum Beispiel Sehzeichen in einer Größe enthalten, die für eine vorbestimmte Prüfentfernung ausgelegt sind. Der Skalierungsfaktor für diese vorab bereitgestellten digitalen Bilder von Prüftafeln kann zum Beispiel in Prozent angegeben werden, um die zugehörigen Bilddaten prozentual zu skalieren, und somit abhängig von der detektierten individuellen Prüfentfernung entweder zu vergrößern oder zu verkleinern.

Als vorbestimmte Regel kann zum Beispiel ein vorbestimmter Winkel dienen, unter dem die Sehzeichen in der individuellen Prüfentfernung dem Probanden erscheinen sollen. Die Abhängigkeit einer solchen Sollgröße von der Prüfentfernung ist zum Beispiel in der europäischen Norm EN ISO 8596:2009 und in der deutschen Norm DIN 58220-5 gegeben. Als vorbestimmte Regel kann somit insbesondere eine genormte Sollgröße dienen, zum Beispiel die nach den aufgeführten Normen genormte Sollgröße für Landoltringe. In den Normen stellt der Erscheinungswinkel der Landoltringe eine vorbestimmte Regel dar, aus der die Größe der Landoltringe für die detektierte individuelle Prüfentfernung als individuelle Sollgröße der Sehzeichen berechnet werden kann.

Nach der Bestimmung der Sollgröße werden digitale Sehzeichenbilddaten bereitgestellt, die auf die individuelle Sollgröße skalierte Sehzeichen enthalten. Die digitalen Sehzeichenbilddaten sind dafür ausgebildet und vorgesehen, auf der Darstellungsoberfläche angezeigt zu werden. Werden die digitalen Sehzeichenbilddaten auf der Darstellungsoberfläche angezeigt, so werden die Sehzeichen in der ihnen zugeordneten, individuellen Sollgröße angezeigt. Von der Probandenposition aus betrachtet weisen die dargestellten Sehzeichen somit genau diejenige Sollgröße auf, die sie gemäß der verwendeten vorbestimmten Regel (zum Beispiel Norm) aufweisen sollen. Damit kann anhand der dargestellten Sehzeichen ein Sehtest durchgeführt werden, insbesondere eine Sehschärfebestimmung für die Ferne. Das Verfahren kann ohne schwerfällige Refraktionseinheiten und ohne klassische, vorgedruckte Prüftafeln durchgeführt werden. Die Sehzeichen können auf eine dynamisch variable, individuelle Prüfentfernung als Prüfabstand abgestimmt werden, insbesondere mit Hilfe eines Computers oder computerähnlichen Geräts.

Gemäß einer Ausführungsform werden das Bestimmen der individuellen Sollgröße für Sehzeichen, das Bereitstellen der digitalen Sehzeichenbilddaten und das Darstellen der digitalen Sehzeichenbilddaten automatisch mittels eines mobilen Computers durchgeführt. Als ein mobiler Computer kann dabei ein Laptop, ein Notebook, ein Tablet-Computer, ein Smartphone oder ein ähnliches elektronisches Gerät verwendet werden, das einen Mikroprozessor aufweist, auf dem ein Betriebssystem läuft, das Programme ausführen kann. Nach z.B. automatischer Detektion der individuellen Prüfentfernung kann der mobile Computer die individuelle Sollgröße in Abhängigkeit der individuellen Prüfentfernung berechnen, und die digitalen Sehzeichenbilddaten bereitstellen. Die generierten digitalen Sehzeichenbilddaten können entweder auf dem Display des Computers angezeigt werden oder an einem Datenausgang des Computers bereitgestellt werden, insbesondere an einem drahtlosen Ausgang.

Der mobile Computer enthält somit alle oder nahezu alle wesentlichen Vorrichtungsgegenstände, die zum Durchführen der Sehschärfebestimmung für die Ferne. Damit ist das Mitführen von Prüftafeln oder einer Refraktionseinheit überflüssig. Dadurch wird die Durchführung des Verfahrens zum Beispiel bei Hausbesuchen und öffentlichen Events vereinfacht und/oder erst ermöglicht.

Alternativ kann auch ein stationärer Computer wie ein Desktop-PC oder Tower-PC verwendet werden, der jedoch komplizierter zu transportieren ist.

Gemäß einer Ausführungsform wird auf der Darstellungsoberfläche ein individueller Testpunktabstand in digitalen Bilddaten bestimmt. Die digitalen Sehzeichenbilddaten werden unter Berücksichtigung des individuellen Testpunktabstands auf die Sollgröße skaliert. Zur Bestimmung des individuellen Testpunktabstands kann auf der Darstellungsoberfläche ein Testbild dargestellt werden, das zumindest zwei prominente Testpunkte enthält, die einfach zu identifizieren sind. Der Abstand dieser Testpunkte voneinander kann entweder manuell oder automatisch ermittelt werden. Durch die Bestimmung des individuellen Testpunktabstands kann festgestellt werden, welchen Abstand zwei Punkte in einem digitalen Bild (also den digitalen Bilddaten) voneinander tatsächlich auf der Darstellungsoberfläche haben. Der Testpunktabstand kann z.B. in einer Längeneinheit wie in Zentimetern angegeben werden. Kenntnis des individuellen Testpunktabstands auf der Darstellungsoberfläche ermöglicht ein Umrechnen von Pixelabständen in digitalen Bilddaten in die tatsächliche Ist-Abstandslänge der korrespondierenden Bildpunkte, die auf der Darstellungsoberfläche angezeigt werden. Der individuelle Testpunktabstand dient zum Kalibrieren der Größe der Sehzeichen, die auf der Darstellungsoberfläche dargestellt werden sollen.

In einer Weiterbildung dieser Ausführungsform wird eine Größe von unskalierten Sehzeichen in unskalierten digitalen Bilddaten unter Berücksichtigung des individuellen Testpunktabstands mittels Dreisatz zum Bereitstellen der digitalen Sehzeichenbilddaten auf die Sollgröße skaliert. Die unskalierten digitalen Bilddaten enthalten dabei Bilder von unskalierten Sehzeichen. Würden diese unskalierten digitalen Bilddaten auf der Darstellungsfläche angezeigt werden, so hätten die angezeigten Sehzeichen nicht die individuelle Sollgröße, es sei denn, sie hätten rein zufällig die richtige Größe. Unter Kenntnis des individuellen Testpunktabstands werden die unskalierten digitalen Bilddaten mittels Dreisatz auf die individuelle Sollgröße gebracht, in der sie in der individuellen Prüfentfernung erscheinen sollen. Dabei werden die digitalen Sehzeichenbilddaten generiert, die in der Regel skaliert und individuell abgestimmt auf die individuelle Prüfentfernung sind. Der Dreisatz bietet eine mathematisch einfache und leicht zu berechnende Möglichkeit, vorbereitete unskalierte digitale Bilddaten auf die individuelle Sollgröße zu skalieren. Alternativ können ähnliche mathematische Verfahren eingesetzt werden, die eine korrekte Skalierung ermöglichen.

Gemäß einer Ausführungsform wird mittels eines Aufnahmegeräts ein digitales Prüfbild aufgenommen, das zum Detektieren der individuellen Prüfentfernung ausgewertet wird. Das digitale Prüfbild kann entweder von der Probandenposition aus über die individuelle Prüfentfernung in Richtung der Darstellungsposition aufgenommen werden, oder von der Darstellungsposition aus in Richtung der Probandenposition. Das digitale Prüfbild wird somit über die individuelle Prüfentfernung hinweg aufgenommen. Als Aufnahmegerät kann zum Beispiel eine Kamera verwendet werden, insbesondere eine Digitalkamera, die automatisch oder halbautomatisch digitale Bilddaten erstellt, die von einem Computer ausgewertet werden können. Bei der Auswertung des digitalen Prüfbilds wird die individuelle Prüfentfernung ermittelt und als ermittelte Variable für das weitere Verfahren bereitgestellt. Alternativ könnte die individuelle Prüfentfernung auch mittels eines anderen Entfernungsdetektors ermittelt werden, und entweder automatisch oder manuell eingegeben werden.

In einer Weiterbildung dieser Ausführungsform weist das Verfahren die Schritte auf:
- Anordnen eines Kalibrierobjekts derart, dass das Kalibrierobjekt vom Aufnahmegerät in der individuellen Prüfentfernung beabstandet ist, und
- Aufnehmen des Prüfbilds derart, dass das Prüfbild eine Aufnahme des in der individuellen Prüfentfernung angeordneten Kalibrierobjekts enthält.

Als Kalibrierobjekt kann dazu ein beliebiges Objekt dienen, bei dem der Abstand zweier prominenter Objektpunkte vorbekannt ist. So kann als Kalibrierobjekt zum Beispiel ein schwarzes Quadrat mit bekannter Seitenlänge dienen, oder ein ähnliches Objekt, bei dem zumindest zwei Objektpunkte (z.B. in einer digitalen Bildauswertung) automatisch erkannt werden können. Bei Aufnahme des Prüfbilds ist das Kalibrierobjekt in der individuellen Prüfentfernung von dem Aufnahmegerät entfernt angeordnet. Dabei kann es insbesondere so angeordnet sein, dass die beabstandeten prominenten Objektpunkte des Kalibrierobjekts senkrecht zum Richtungsvektor der Prüfentfernung von der Probandenposition zur Darstellungsposition ausgerichtet sind. Im Beispiel eines flächigen schwarzen Quadrats könnte dieses flächige schwarze Quadrat zum Beispiel flach auf die Darstellungsoberfläche gehalten werden, und das Prüfbild ein Bild der Darstellungsoberfläche sein, auf dem das Kalibrierobjekt angeordnet ist. Zumindest zwei prominente Objektpunkte des Kalibrierobjekts werden im Prüfbild entweder automatisch oder manuell bei einer Bildauswertung identifiziert und markiert. Aus der Größe des Abstands der prominenten Objektpunkte des Kalibrierobjekts im digitalen Prüfbild wird die individuelle Prüfentfernung berechnet und dadurch bestimmt.

In einer Weiterbildung dieser Ausführungsform wird als Darstellungsoberfläche eine computergesteuerte Projektionsfläche verwendet und das Prüfbild von der Probandenposition aus so aufgenommen, dass das Prüfbild zumindest teilweise eine Aufnahme der Projektionsfläche enthält. Dabei bedeutet computergesteuert, dass auf der Projektionsfläche digitale Daten angezeigt werden können. Als solche Projektionsfläche kann zum Beispiel ein an einer Wand angeordneter Display, ein freistehender Monitor, eine Leinwand (z.B. für einen Projektor), eine Zimmerwand (z.B. für einen Beamer), etc. verwendet werden. Auf der Projektionsfläche können computergenerierte Bilddaten dargestellt werden, insbesondere die bereitgestellten digitalen Sehzeichenbilddaten. Das Prüfbild enthält dabei zumindest teilweise eine Aufnahme der Projektionsfläche.

In einer Weiterbildung dieser Ausführungsform wird zur Bestimmung des individuellen Testpunktabstands ein auf die Projektionsfläche projiziertes Testbild ausgewertet. Dabei kann eine Aufnahme des projizierten Testbilds gemacht werden, insbesondere eine digitale Aufnahme mit dem Aufnahmegerät. Zur Bestimmung des individuellen Testpunktabstands kann insbesondere das digitale Prüfbild verwendet werden, das zur Bestimmung der individuellen Prüfentfernung ausgewertet wird. So kann unter Auswertung eines einzigen Prüfbilds sowohl die individuelle Prüfentfernung ermittelt und/oder berechnet werden, als auch der individuelle Testpunktabstand in digitalen Bilddaten. In diesem Fall kann das digitale Prüfbild sowohl das Testbild als auch das Kalibrierobjekt enthalten. Als eine besonders einfache Ausführungsform des Testbilds kann zum Beispiel ein einfarbiges, gesamtes Ausleuchten der Projektionsfläche dienen. Dadurch kann die Größe der gesamten Projektionsfläche in den aufgenommenen Bilddaten gemessen, ermittelt und/oder bereitgestellt werden, aus der der individuelle Testpunktabstand bestimmt werden kann. Das Testbild kann zumindest zwei prominente Bildpunkte enthalten, die zum Beispiel bei einer digitalen Bildauswertung schnell und sicher (manuell oder automatisch) erkannt werden.

In einer alternativen Ausführungsform wird als Darstellungsoberfläche das Display eines Computers verwendet und das Prüfbild von einer computerinternen Digitalkamera des Computers aufgenommen. Insbesondere Tablet-Computer oder Laptops weisen häufig computerinterne Digitalkameras auf, die zum Detektieren der individuellen Prüfentfernung genutzt werden können. Insbesondere kann diejenige computerinterne Digitalkamera verwendet werden, die auf der Höhe und auf der Seite des Computerdisplays angeordnet ist. Diese computerinterne Digitalkamera ist somit in dieser Ausführungsform an der Darstellungsposition und/oder an einer vorbekannten festen Position relativ zur Darstellungspsition angeordnet. Als Prüfbild kann somit von der computerinternen Digitalkamera eine Aufnahme des Probanden gemacht werden, der an der Probandenposition angeordnet ist.

Auch in der oben beschriebenen Ausführungsform, in der eine zum Beispiel externe Projektionsfläche als Darstellungsoberfläche verwendet wird, kann das Prüfbild mittels einer computerinternen Digitalkamera aufgenommen werden, wodurch dem Computer unmittelbar und sehr schnell das Prüfbild zur weiteren Verarbeitung zur Verfügung gestellt wird.

In einer Weiterbildung der Ausführungsform mit dem Display als Darstellungsoberfläche kann als Kalibrierobjekt eine Refraktionsbrille verwendet werden, die der Proband beim Aufnehmen des Prüfbilds trägt. Zur Durchführung des geplanten Sehtests ist üblicherweise sowieso eine Refraktionsbrille nötig, die der Proband bei Durchführung des Sehtests trägt. Zur Detektion der Prüfentfernung können zwei prominente Objektpunkte an der Refraktionsbrille verwendet werden, deren Abstand zueinander während des Kalibriervorgangs fixiert und somit nicht variabel ist. Dazu kann zum Beispiel eine handelsübliche Refraktionsbrille verwendet werden, bei der solch prominente Objektpunkte leicht erkennbar sind und/oder es können spezielle Markierungen an einer beliebigen Refraktionsbrille angeordnet werden. Als Objektpunkte können dabei statische Punkte der Refraktionsbrille verwendet werden, deren Abstand zueinander unabhängig von einer aktuellen Einstellung der Refraktionsbrille wie einem Pupillenabstand etc. ist. Der Abstand der Markierungen zueinander ist vorbekannt und kann im digitalen Prüfbild entweder manuell oder automatisch markiert und/oder ermittelt werden. Aus dem Abstand der Markierungen bzw. Objektpunkte der Refraktionsbrille im digitalen Prüfbild lässt sich der individuelle Prüfabstand ermitteln.

In einer Weiterbildung der Ausführungsform werden die digitalen Sehzeichenbilddaten unter Berücksichtigung der Pixelgröße des Displays als individueller Testpunktabstand auf die individuelle Sollgröße skaliert. Da in dieser Ausführungsform der Display des Computers als Darstellungsoberfläche verwendet wird, muss der individuelle Testpunktabstand nicht unbedingt eigens ermittelt werden, sondern kann zum Beispiel durch den Hersteller des Computers vorgegeben sein. Zur Kontrolle kann der Pixelabstand auch nachgemessen werden. Bei Verwendung des Displays des Computers muss der individuelle Testpunktabstand lediglich einmal ermittelt werden, wobei der Computer selbst als Vorrichtung zur Durchführung des Verfahrens dient, der in der individuellen Prüfentfernung vom Probanden beabstandet aufgestellt wird.

Gemäß einer Ausführungsform wird eine Umgebungshelligkeit erfasst und beim Darstellen der digitalen Sehzeichenbilddaten die Helligkeit der dargestellten Sehzeichen und/oder die Helligkeit der unmittelbare Umgebung der dargestellten Sehzeichen auf der Darstellungsoberfläche an die erfasste Umgebungshelligkeit angepasst. So kann z.B. die Displayhelligkeit angepasst werden, so dass bei der Sehanalyse störende Einflüsse wie Blendung und/oder Verfälschungen durch Überblendung von Sehzeichen sowie Streueffekte an brechenden Medien (z.B. Refraktionsgläsern) reduziert werden. Dabei kann der Kontrast und/oder die Helligkeit der dargestellten Sehzeichen und/oder deren Umgebung an genormte Bezugswerte angepasst werden, die z.B. in den weiter oben zitierten Normen angegeben sind.

In einer Ausführungsform beträgt die individuelle Prüfentfernung von einem Meter bis zehn Meter. Besonders bevorzugt wird eine individuelle Prüfentfernung von vier Meter bis sechs Meter verwendet. Dabei kann für jede Messung eine unterschiedliche Prüfentfernung verwendet und eingestellt werden.

Gemäß einer Ausführungsform wird das Verfahren für unterschiedliche individuelle Prüfentfernungen durchgeführt und für jede individuelle Prüfentfernung digitale Sehzeichenbilddaten bereitgestellt, die auf die individuelle Sollgröße skalierte Sehzeichen enthalten. Mit ein und derselben Vorrichtung kann eine Sehschärfebestimmung für die Ferne durchgeführt werden, die auf eine individuelle, dynamisch variable Prüfentfernung abgestimmt ist.

Ein Aspekt betrifft ein Verfahren zum Bestimmen von optischen Korrekturwerten eines Probanden, bei dem Sehzeichen für eine Sehschärfebestimmung für die Ferne nach dem vorangehend beschriebenen Aspekt bereitgestellt werden, wobei der Proband die bereitgestellten Sehzeichen durch Besichtigung von der Probandenposition aus zu erkennen versucht und das Ergebnis der Besichtigung in einen Computer eingegeben werden. Dies ermöglicht eine automatische Auswertung der Sehschärfebestimmung mittels des Computers. Dazu kann zum Beispiel ein mobiler Computer verwendet werden. Als Computer dient insbesondere der Computer, mit dem das Detektieren der individuellen Prüfentfernung usw. durchgeführt wird. Die Eingabe erfolgt über ein Eingabeinterface des Computers, also zum Beispiel über eine Tastatur und/oder über einen Touchscreen. Das Ergebnis kann entweder vom Probanden selbst in den Computer eingegeben werden, einem Prüfer diktiert werden und/oder mittels einer Fernbedienung eingegeben werden. Der Computer kann anschließend eine Auswertung des Tests zur Sehschärfebestimmung anzeigen, insbesondere zum Visus, dem Kontrastvermögen, der Akkommodationsfähigkeit, dem Farbensehen und/oder dem Binokularstatus des Probanden. Das Verfahren kann weiterhin zusätzliche Sehtests enthalten, z.B. eine Sehschärfebestimmung für die Nähe.

Ein Aspekt betrifft ein Computerprogrammprodukt, das Programmteile aufweist, welche, wenn in einen Computer geladen, zur Durchführung eines computerimplementierten Verfahrens nach einem der vorangehend beschriebenen Aspekten ausgelegt sind. Das Computerprogrammprodukt kann zum Beispiel ein Computerprogramm sein, eine App und/oder ein digitaler Speicher, auf dem das Computerprogrammprodukt gespeichert ist. Das Computerprogrammprodukt kann dabei insbesondere dazu ausgelegt und vorgesehen sein, die folgenden Schritte des Verfahrens nach einem der vorangehend beschriebenen Aspekte durchzuführen:
- Detektieren einer individuellen Prüfentfernung zwischen der Probandenposition und der Darstellungsposition,
- Bestimmen einer individuellen Sollgröße für Sehzeichen, die gemäß einer vorbestimmten Regel auf die detektierte individuelle Prüfentfernüng abgestimmt sind,
- Bereitstellen von digitalen Sehzeichenbilddaten, die auf die individuelle Sollgröße skalierte Sehzeichen enthalten, und
- Darstellen der digitalen Sehzeichenbilddaten auf der Darstellungsoberfläche.

Vorangehende Verfahrensschritte, wie z.B. das Bereitstellen der Probandenposition und der Darstellungsoberfläche an der Darstellungsposition, können von einer Bedienperson durchgeführt werden. Das Computerprogrammprodukt dient somit insbesondere zur Unterstützung der Durchführung des Verfahrens durch eine Bedienperson.

Dabei kann das Computerprogrammprodukt insbesondere dazu ausgebildet und vorgesehen sein, mittels eines Aufnahmegeräts ein digitales Prüfbild aufzunehmen, das z.B. automatisch oder halbautomatisch zum Detektieren der individuellen Prüfentfernung ausgewertet wird.

Dabei kann von einer Bedienperson ein Kalibrierobjekt derart angeordnet werden, dass das Kalibrierobjekt vom Aufnahmegerät in der individuellen Prüfentfernung beabstandet ist.

Das Computerprogrammprodukt kann dazu ausgebildet und vorgesehen sein, das Prüfbilds derart aufzunehmen, dass das Prüfbild eine Aufnahme des in der individuellen Prüfentfernung angeordneten Kalibrierobjekts enthält.

Ein Aspekt betrifft eine Vorrichtung zur Durchführung des voranstehend beschriebenen Verfahrens. Eine solche Vorrichtung kann z.B. einen mobilen Computer mit einer computerinternen Digitalkamera aufweisen, der ein Computerprogrammprodukt gemäß dem vorangehenden Aspekt aufweist.

Die Erfindung wird nachfolgend anhand von einzelnen Merkmalen einiger Ausführungsbeispiele ohne bevorzugte Reihenfolge näher beschrieben.

Durch die Gegenstände der unabhängigen Ansprüche wird eine Sehschärfebestimmung für eine variable Prüfentfernung und für eine Vielzahl von unterschiedlichen Sehtests bereitgestellt. Eine Sehschärfebestimmung kann sowohl in einem Refraktionsraum bei einem Arzt, Augenoptiker etc. durchgeführt werden, als auch bei einem Hausbesuch, in Krankenhäusern und bei Events.

Es besteht die Möglichkeit, dass der Proband selber die von ihm erkannten Sehzeichen an einem Bedienelement auswählen und eingeben kann, woraufhin die Eingabe automatisch auf Richtigkeit hin überprüft werden kann. Dadurch ist z.B. ein prüfender Augenoptiker selbst nicht auf einen guten Visus angewiesen, da ihm die automatische Abfrage über die Erkennung von Sehzeichen erleichtert wird.

Wird die Erfindung mittels eines Computers durchgeführt, so kann der Computer eine Schnittstelle zu einer Branchensoftware aufweisen. Über die Schnittstelle können ermittelte Refraktionsdaten automatisch oder manuell an die Branchensoftware übermittelt werden. Dabei können z.B. Brillengläser bestellt werden und/oder deren Verfügbarkeit und/oder Preis abgefragt werden.

Durch die vorgeschlagene Lösung können kostenintensive, nicht transportable Sehtestgeräte ersetzt werden. Es wird eine All-In-One Alternative zu bislang bekannten Sehtestgeräten bereitgestellt, die aufgrund ihrer mobilen Einsetzbarkeit und kostengünstigen Anschaffung auch z.B. in Entwicklungsländern genutzt werden kann.

Eine Ausführungsform betrifft eine Vorrichtung zum Durchführen einer Sehschärfebestimmung mit einem mobilen Computer. Der mobile Computer weist eine Eingabeeinheit, eine visuelle Ausgabeeinheit und eine Digitalkamera auf. Die Vorrichtung kann mit externen Display und/oder einer von einem Projektor angesteuerten Projektionsfläche als Darstellungsoberfläche gekoppelt werden. In diesem Fall werden die auf der Darstellungsoberfläche angezeigten Inhalte über die Ein- und/oder Ausgabeeinheit des mobilen Computers gesteuert und/oder geregelt. Alternativ oder zusätzlich kann der mobile Computer alleine verwendet werden, wobei die visuelle Ausgabeeinheit (wie z.B. ein Display) als Darstellungsoberfläche dient. Ein auf dem mobilen Computer installiertes Computerprogrammprodukt kann eine entsprechende Auswahl der Darstellungsoberfläche enthalten oder für eine Vorbestimmte der beiden Alternativen ausgebildet und vorgesehen sein.

Eine Eingabeeinheit des Computers kann zur Steuerung der Anzeigeinhalte und/oder zur Rückmeldung über durchgeführte Testaufgaben verwendet werden.

In einer Ausführungsform wird ein Kalibrierobjekt mit zumindest teilweise bekannten Abmessungen zur Bestimmung der Prüfentfernung verwendet. Dabei wird ein digitales Prüfbild vom Kalibrierobjekt aufgenommen, das dabei in der Darstellungsebene angeordnet ist. Aus den zumindest teilweise bekannten Abmessungen des Kalibrierobjekts im digitalen Prüfbild kann der individuelle Testpunktabstand bestimmt werden, also z.B. der tatsächliche Ist-Abstand zweier Bildpunkte auf einer computergesteuerten Projektionsfläche.

Bei Nutzung der für die Sehschärfebestimmung verwendeten Refraktionsbrille als Kalibrierobjekt kann berücksichtigt werden, dass die Refraktionsbrille individuell auf den Probanden einstellbar ist. Beispielsweise kann die Refraktionsbrille auf den individuellen Pupillenabstand des Probanden eingestellt werden. Als wohldefinierte, ausgezeichnete und/oder prominente Punkte der Refraktionsbrille als Kalibrierobjekt können Markierungen zum Einstellen des Pupillenabstands verwendet werden, wie z.B. ein innerer Rand der Glashalterungen, oder speziell angebrachte Elemente wie z.B. blaue Kugeln bei der Oculus UB-4 Refraktionsbrille.

Mittels einer festgelegten Kalibrierung, die einen Zusammenhang zwischen der tatsächlichen Objektentfernung vom Aufnahmegerät einerseits und dem Abstand in Pixeln im Prüfbild des Aufnahmegeräts andererseits angibt, kann der Abstand des Kalibrierobjekts von dem Aufnahmegerät ermittelt werden, welcher der individuellen Prüfentfernung entspricht.

Als Kalibrierobjekt kann ein kontrastreicher Ausdruck verwendet werden, wie z.B. ein schwarzes geometrisches Objekt (z.B. Quadrat), das von einem weißen Rand umgeben ist. Zur Aufnahme des oben beschriebenen Prüfbilds kann der Ausdruck an der Darstellungsoberfläche befestigt werden.

Zur Kalibrierung kann von dem Kalibrierobjekt in mehreren definierten Entfernungen vom Aufnahmegerät aus jeweils zumindest eine Kalibrieraufnahme aufgenommen werden. Aus den Pixelabständen prominenter Objektpunkte in den jeweiligen Kalibrieraufnahmen kann ein Zusammenhang zwischen den definierten Entfernungen und der Größe des Kalibrierobjekts in den mit dem Aufnahmegerät aufgenommen Bildern hergestellt werden. Mit Hilfe dieser Kalibrierung, also diesem Zusammenhang, kann aus dem digitalen Prüfbild, das eine Aufnahme des Kalibrierobjekts enthält, die individuelle Prüfentfernung ermittelt werden.

Bei der Sehschärfebestimmung werden dem Probanden die Sehzeichen gezeigt. Der Proband versucht, die Sehzeichen korrekt zu erkennen. Diese vom Probanden erkannte Lösung der mittels der Sehzeichen bereitgestellten Sehtests, wie z.B. die Richtung der Öffnung bei Landolt-Ringen, kann entweder vom Proband oder einer Prüfperson über ein Eingabeinterface des Computers rückgemeldet werden. Aus dieser Rückmeldung kann anhand eines vom Computer durchgeführten Vergleichs mit den dargestellten Sehaufgaben ermittelt werden, ob die Sehaufgaben korrekt gelöst wurden, also z.B. die Sehzeichen korrekt vom Probanden erkannt wurden. Aus einer Mehrzahl solcher Rückmeldungen kann eine Aussage über die Sehfähigkeit des Probanden getroffen werden, wie z.B. dessen Visus, dessen Kontrastsehen, etc. Dazu kann z.B. eine automatisierte Abfolge von Sehaufgaben verwendet und/oder bereitgestellt werden.

Dadurch können Sehzeichen und Testinhalte entfernungsunabhängig und variabel in der Größe dargestellt werden. Durch eine einfache und schnelle Kalibrierung wird die Applikation an die örtlichen Gegebenheiten angepasst um einen Sehtest z.B. in normgerechter Darstellung durchzuführen.

Dabei kann ein mobiler Computer als Bedienelement dienen. Der mobile Computer alleine kann als Refraktionseinrichtung verwendet werden, gegebenenfalls zusammen mit einer Fernbedienung zur Eingabe der Rückmeldungen des Probanden. Dabei kann der mobile Computer in einer beliebigen Prüfentfernung vom Probanden aufgestellt werden, bevorzugt jedoch in einer beliebigen Prüfentfernung von 1 m bis 10 m.

Der mobile Computer kann als mobile Refraktionseinheit verwendet werden und/oder als Softwareträger für ein Projektionsgerät, das als lokale Einheit am Prüfort angeordnet sein kann. Dadurch wird sowohl ein lokaler als auch ein zusätzlicher mobiler Einsatz des mobilen Computers für eine stationäre und mobile Refraktion mit denselben Kernelementen ermöglicht.

Für das verwendete Aufnahmegerät kann eine Korrektur der Abbildungsfehler der verwendeten Kameraoptik ermittelt werden, die bei der Aufnahme von Bildern (Prüfbild, Kalibrieraufnahme, etc.) korrigiert werden kann.

## Patentansprüche

1. Verfahren zum Bereitstellen von Sehzeichen für eine Sehschärfebestimmung für die Ferne, mit den Schritten:
- Bereitstellen einer Probandenposition für einen Probanden,
- Bereitstellen einer Darstellungsoberfläche an einer Darstellungsposition,
- Detektieren einer individuellen Prüfentfernung zwischen der Probandenposition und der Darstellungsposition,
- Bestimmen einer individuellen Sollgröße für Sehzeichen, die gemäß einer vorbestimmten Regel auf die detektierte individuelle Prüfentfernung abgestimmt sind,
- Bereitstellen von digitalen Sehzeichenbilddaten, die auf die individuelle Sollgröße skalierte Sehzeichen enthalten, und
- Darstellen der digitalen Sehzeichenbilddaten auf der Darstellungsoberfläche;
**dadurch gekennzeichnet, dass** mittels eines Aufnahmegeräts ein digitales Prüfbild aufgenommen wird, das zum Detektieren der individuellen Prüfentfernung ausgewertet wird, mit den Schritten:
- Anordnen eines Kalibrierobjekts derart, dass das Kalibrierobjekt vom Aufnahmegerät in der individuellen Prüfentfernung beabstandet ist,
- Aufnehmen des Prüfbilds derart, dass das Prüfbild eine Aufnahme des in der individuellen Prüfentfernung angeordneten Kalibrierobjekts enthält und
- Berechnen und Bestimmen der individuellen Prüfentfernung aus der Größe eines Abstands zumindest zweier prominenter Objektpunkte des Kalibrierobjekts im digitalen Prüfbild.

2. Verfahren nach Anspruch 1, wobei das Bestimmen der individuellen Sollgröße für Sehzeichen, das Bereitstellen der digitalen Sehzeichenbilddaten und das Darstellen der digitalen Sehzeichenbilddaten automatisch mittels eines mobilen Computers durchgeführt werden.

3. Verfahren nach Anspruch 1 oder 2, wobei auf der Darstellungsoberfläche ein individueller Testpunktabstand in digitalen Bilddaten bestimmt wird, und die digitalen Sehzeichenbilddaten unter Berücksichtigung des individuellen Testpunktabstands auf die Sollgröße skaliert werden.

4. Verfahren nach Anspruch 3, wobei eine Größe von unskalierten Sehzeichen in unskalierten digitalen Bilddaten unter Berücksichtigung des individuellen Testpunktabstands mittels Dreisatz zum Bereitstellen der digitalen Sehzeichenbilddaten auf die Sollgröße skaliert wird.

5. Verfahren nach einem der vorangegangenen Ansprüche, wobei als Darstellungsoberfläche eine computergesteuerte Projektionsfläche verwendet wird und das Prüfbild von der Probandenposition aus so aufgenommen wird, dass das Prüfbild eine Aufnahme der Projektionsfläche enthält.

6. Verfahren nach Anspruch 3 und 5, wobei ein auf die Projektionsfläche projiziertes Testbild zur Bestimmung des individuellen Testpunktabstands ausgewertet wird.

7. Verfahren nach einem der vorangegangenen Ansprüche, wobei als Darstellungsoberfläche das Display eines Computers verwendet wird und das Prüfbild von einer computerinternen Digitalkamera des Computers aufgenommen wird.

8. Verfahren nach Anspruch 7, wobei als Kalibrierobjekt eine Refraktionsbrille verwendet wird, die der Proband beim Aufnehmen des Prüfbilds trägt.

9. Verfahren nach Anspruch 7 oder 8, wobei die digitalen Sehzeichenbilddaten unter Berücksichtigung der Pixelgröße des Displays als individueller Testpunktabstand auf die individuelle Sollgröße skaliert werden.

10. Verfahren nach einem der vorangegangenen Ansprüche, wobei eine Umgebungshelligkeit erfasst wird und beim Darstellen der digitalen Sehzeichenbilddaten die Helligkeit der dargestellten Sehzeichen und/oder die Helligkeit der unmittelbare Umgebung der dargestellten Sehzeichen auf der Darstellungsoberfläche an die erfasste Umgebungshelligkeit angepasst wird.

11. Verfahren nach einem der vorangegangenen Ansprüche, wobei die individuelle Prüfentfernung von 1 m bis 10 m beträgt.

12. Verfahren nach einem der vorangegangenen Ansprüche, wobei das Verfahren für unterschiedliche individuelle Prüfentfernungen durchgeführt wird und für jede individuelle Prüfentfernung digitale Sehzeichenbilddaten bereitgestellt werden, die auf die individuelle Sollgröße skalierte Sehzeichen enthalten.

13. Verfahren zum Bestimmen von optischen Korrekturwerten eines Probanden, bei dem Sehzeichen für eine Sehschärfebestimmung für die Ferne nach einem der vorangegangenen Ansprüche bereitgestellt werden, wobei der Proband die bereitgestellten Sehzeichen durch Besichtigung von der Probandenposition aus zu erkennen versucht und das Ergebnis der Besichtigung in einen Computer eingegeben werden.

14. Computerprogrammprodukt aufweisend Programmteile, welche, wenn in einem Computer mit einer computerinternen Digitalkamera geladen, zur Durchführung eines computer-implementierten Verfahrens nach einem der vorangegangenen Ansprüche ausgelegt sind.

15. Mobiler Computer mit einer computerinternen Digitalkamera, der ein Computerprogrammprodukt nach Anspruch 14 aufweist.

## Claims

1. A method for providing optotypes for a visual acuity determination for the distance, with the steps:
- Providing a subject position for a subject,
- Providing a presentation surface at a presentation position,
- Detecting an individual test distance between the subject position and the presentation position,
- Determining an individual target value for optotypes, which are tuned to the detected individual test distance according to a predetermined rule,
- Providing digital optotype image data containing optotypes, which are scaled on the individual target size, and
- Displaying the digital optotype image data on the display surface;
**characterized in that** by means of a recording device, a digital test image is recorded, which is evaluated for detecting the individual test distance, comprising the steps:
- Arranging a calibration object such that the calibration object is spaced from the recording device at the individual test distance,
- Recording the test image such that the test image contains a recording of the calibration object, which is arranged in the individual test distance, and
- Calculating and determining the individual test distance from the size of a distance of at least two prominent object points of the calibration object in the digital test image.

2. The method of claim 1, wherein determining the individual target size for optotypes, providing the digital optotype image data, and displaying the digital optotype image data is performed automatically by a mobile computer.

3. The method of claim 1 or 2, wherein on the display surface, an individual test point distance in digital image data is determined, and the digital optotype image data are scaled to the target size taking into account the individual test point distance.

4. The method of claim 3, wherein a size of unscaled optotypes in unscaled digital image data is scaled to the target size by taking into account the individual test point distance by means of the rule of three for providing the digital optotype image data.

5. The method according to any one of the preceding claims, wherein a computer-controlled projection surface is used as a display surface and the test image is taken from the subject position so that the test image contains a recording of the projection surface.

6. The method of claim 3 and 5, wherein a test image, which is projected onto the projection surface is evaluated to determine the individual test point distance.

7. The method according to any one of the preceding claims, wherein the display surface of a computer is used as the display surface and the test image is taken from a computer-internal digital camera of the computer.

8. The method of claim 7, wherein a pair of refraction glasses is used as a calibration object, which the subject is wearing when recording the test pattern.

9. The method of claim 7 or 8, wherein the digital optotype image data are scaled to the individual target size, taking into account the pixel size of the display as an individual test point distance.

10. The method according to any one of the preceding claims, wherein an ambient brightness is detected and when displaying the digital optotype image data, the brightness of the displayed optotypes and / or the brightness of the immediate surroundings of the displayed optotypes on the presentation surface are adapted to the detected ambient brightness.

11. A method according to any one of the preceding claims, wherein the individual test distance is from 1 m to 10 m.

12. A method according to any one of the preceding claims, wherein the method is performed for different individual test distances, and for each individual test distance, digital optotype image data are provided, which include optotypes scaled to the individual desired size.

13. A method for determining optical correction values of a subject, in which optotypes for a visual acuity determination for the distance according to one of the preceding claims are provided, wherein the subject tries to recognize the provided optotypes by viewing from the subject position and the result of the inspection is entered into a computer.

14. A computer program product comprising program parts which, when loaded in a computer having a computer-internal digital camera, are adapted to carry out a computer-implemented method according to one of the preceding claims.

15. A mobile computer having an computer-internal digital camera having a computer program product according to claim 14.

## Revendications

1. Une méthode pour fournir des optotypes pour la détermination de l'acuité visuelle de la distance, comprenant les étapes suivantes:
- Fournir une position de sujet pour un sujet,
- Fournir une surface de présentation à une position de présentation,
- Détecter une distance de test individuelle entre la position du sujet et la position de présentation,
- Déterminer une valeur cible individuelle pour les optotypes, qui sont réglés sur la distance de test individuelle détectée conformément à une règle prédéfinie,
- Fournir des données d'image d'optotype numériques contenant des optotypes, qui sont adaptés à la taille de la cible, et
- Affichage des données d'image d'optotype numérique sur la surface d'affichage;
**caractérisé en ce que**, au moyen d'un dispositif d'enregistrement, une image de test numérique est enregistrée, laquelle examine la distance de test individuelle, comprenant les étapes consistant à:
- Disposer un objet à étalonner de telle sorte qu'il soit éloigné de l'appareil d'enregistrement à la distance de test individuelle,
- Enregistrer l'image test de telle sorte que l'image test contient un enregistrement de l'objet à étalonner, situé dans la distance de test individuelle, et
- Calculer et déterminer la distance de test individuelle à partir de la distance de deux points d'objet saillants de l'objet à étalonner dans l'image de test numérique.

2. Procédé selon la revendication 1, dans lequel la détermination de la taille de la cible individuelle pour les optotypes, fournir les données d'image d'optotype numérique et l'affichage des données d'image d'optotype numériques sont fait par un ordinateur mobile.

3. Procédé selon la revendication 1 ou 2, dans lequel une distance de point de test individuel dans des données d'image numériques est déterminée sur la surface d'affichage, et les données d'image d'optotype numérique sont mise à l'échelle à la taille cible.

4. Le procédé de la revendication 3, dans lequel une taille des données d'images numériques non mises à l'échelle est mise à l'échelle à la taille cible en tenant compte de la distance du point de test individuel au moyen de la règle de trois pour fournir les données d'image à optotype numérique.

5. Procédé selon l'une quelconque des revendications précédentes, dans lequel une surface de projection commandée par ordinateur est utilisée comme surface d'affichage et l'image de test est prise à partir de la position du sujet de sorte que l'image de test contienne un enregistrement de la surface de projection.

6. Procédé selon la revendication 3 et 5, dans lequel une image de test projetée sur la surface de projection est évaluée pour déterminer la distance de point de test individuel.

7. Procédé selon l'une quelconque des revendications précédentes, dans lequel la surface d'affichage d'un ordinateur est utilisée comme surface d'affichage et l'image de test est prise à partir d'un appareil photo numérique interne de l'ordinateur.

8. Procédé selon la revendication 7, dans lequel, en tant qu'objet d'étalonnage, on utilise un verre de réfraction que le sujet porte en cours de l'enregistrement du motif de test.

9. Procédé selon la revendication 7 ou 8, dans lequel les données d'image d'optotype numérique doivent être mis à l'échelle en tenant compte de la taille en pixels de l'affichage en tant que distance de point de test individuel par rapport à la taille de cible individuelle.

10. Procédé selon l'une quelconque des revendications précédentes, dans lequel une luminosité ambiante est détectée et lors de l'affichage des données d'image d'optotype numérique, la luminosité des optotypes affichés et / ou la luminosité de l'environnement immédiat des optotypes affichés sur la surface de présentation sont adaptées à la luminosité ambiante détectée.

11. Procédé selon l'une quelconque des revendications précédentes, dans lequel la distance de test individuelle est comprise entre 1 m et 10 m.

12. Procédé selon l'une quelconque des revendications précédentes, dans lequel le procédé est exécuté pour différentes distances de test individuelles, et pour chaque distance de test individuelle, des données d'image numériques d'optotype sont fournies, lesquelles comprennent des optotypes mis à l'échelle à la taille individuelle souhaitée.

13. Procédé pour déterminer les valeurs de correction optique d'un sujet, dans lequel des optotypes pour la détermination de l'acuité visuelle de la distance selon l'une quelconque des revendications précédentes sont fourni, dans lequel le sujet essaie de reconnaître les optotypes fournis en regardant à partir de la position du sujet et le résultat de l'inspection est entré dans un ordinateur.

14. Produit programme d'ordinateur comprenant des parties de programme qui, lorsqu'elles sont chargées dans un ordinateur ayant un appareil photo numérique interne, sont adaptées pour mettre en oeuvre un procédé mis en oeuvre par ordinateur selon l'une des revendications précédentes.

15. Ordinateur mobile ayant un appareil photo numérique interne ayant un produit programme d'ordinateur selon la revendication 14.
